# FASCICULE DE BREVET EUROPEEN

(11) **EP 1 406 589 B1**
(45) Date de publication et mention de la délivrance du brevet: **07.09.2016**
(21) Numéro de dépôt: 02767553.7
(22) Date de dépôt: 08.07.2002
(51) Int. Cl.: A61K 8/64, A61Q 19/00, A61Q 19/08, A61Q 5/00

(54) **UTILISATION COSMETIQUE OU DERMATOLOGIQUE DE PEPTIDES POUR AUGMENTER L'ADHESION ENTRE LES CELLULES CUTANEES**
KOSMETISCHE ODER DERMATOLOGISCHE VERWENDUNG VON PEPTIDEN ZUR VERBESSERUNG DER ADHÄSION ZWISCHEN HAUTZELLEN
COSMETIC OR DERMATOLOGICAL USE OF PEPTIDES FOR PROMOTING ADHESION BETWEEN SKIN CELLS

(30) Priorité: 13.07.2001 FR 0109366
(43) Date de publication de la demande: 14.04.2004
(73) Titulaire: Ashland Specialties France, 06410 Biot (FR)
(72) Inventeur: DAL FARRA, Claude, F-06650 Opio (FR); DOMLOGE, Nouha, F-06560 Valbonne (FR)
(74) Mandataire: Macquet, Christophe
(86) Numéro de dépôt international: PCT/FR2002/002380
(87) Numéro de publication internationale: WO 2003/007905

(56) Documents cités:
- EP-A- 0 764 444
- EP-A2- 0 366 638
- WO-A-95/17204
- WO-A2-01/91700
- WO-A2-02/16408
- WO-A2-2007/057449
- DE-A- 4 244 418
- FR-A- 2 784 029
- US-A- 5 380 668
- US-A- 5 849 323
- DATABASE CA [en ligne] CHEMICAL ABSTRACTS SERVICE, COLUMBUS, OHIO, US; Database accession no. 1983:438803 XP002198285 & K.D. KOPPLE ET AL.: "Synthesis and backbone conformations of cyclic hexapeptides cyclo(X-Pro-D-Gln)2" INT. J. PEPT. PROTEIN RESEARCH, vol. 21, no. 3, 1983, pages 269-280,
- DATABASE WPI Week 199734 Derwent Publications Ltd., London, GB; AN 1997-369468 XP002198286 & JP 09 157291 A (Y. SUETSUNA), 17 juin 1997 (1997-06-17)
- KOPPLE KENNETH D ET AL: "Synthesis and backbone conformations of cyclic hexapeptides cyclo(X-Pro-D-Gln)2", INTERNATIONAL JOURNAL OF PEPTIDE AND PROTEIN RESEARCH, MUNKSGAARD, COPENHAGEN, DK, vol. 21, no. 3, 1 January 1983 (1983-01-01), pages 269-280, XP009126048, ISSN: 0367-8377

## Description

La présente invention se rapporte à l'utilisation pour la préparation d'une composition cosmétique ou dermatologique, d'une quantité efficace de peptides de séquence (Gly-Pro-Gln)ₙ-NH₂, dans laquelle n est compris entre 1 et 3, et où les acides aminés peuvent être sous la forme L, D ou DL ; les peptides ou la composition étant destinés à :
- augmenter l'adhésion entre les cellules cutanées,
- augmenter l'adhésion cellulaire,
- traiter de manière curative et/ou préventive les manifestations cutanées du
vieillissement (d'origine physiologique et solaire) et améliorer l'aspect de la peau. Selon un mode de réalisation actuellement préféré de l'invention, le peptide précité est de séquence (Gly-Pro-Gln)₂-NH₂.

Le vieillissement cutané est un phénomène complexe qui est dû à de nombreux facteurs endogènes et exogènes. Cliniquement, on observe l'apparition de rides et ridules, une perte de l'élasticité cutanée, un relâchement des tissus cutanés et sous-cutanés...
De nombreuses voies de recherche sont proposées pour lutter contre le vieillissement, parmi lesquelles la protection contre l'environnement (soleil, pollutions...), l'activation de la régénération cellulaire, le renforcement de la matrice extracellulaire (collagène et élastine). Récemment, des études ont montré l'importance de l'adhésion kératinocytes - jonction dermo-épidermique dans le traitement des peaux âgées.
De façon générale, en augmentant l'adhésion des cellules entre elles, et l'adhésion entre les cellules et la matrice extra-cellulaire, il est possible de prévenir, voire de traiter le relâchement de la peau.
Cette dernière voie de recherche, prometteuse, est encore peu étudiée, alors que le rapprochement cellule-matrice ne peut que favoriser les échanges que l'on sait maintenant nombreux entre la cellule et son milieu environnant complexe.

Elément principal de la matrice extra-cellulaire, le collagène participe à la structure, à la consistance de la peau. Très étudié pour ses nombreuses fonctions, il est aussi abondamment utilisée en cosmétique.

Bien que son utilisation se soit réduite depuis les problèmes de l'ESB, le collagène demeure très employé notamment au travers des collagènes marins (natifs et hydrolysats). L'industrie cosmétique récemment sensibilisée à l'utilisation des peptides en biologie cutanée (séquences dérivées de l'alpha-MSH, de certains neuropeptides comme le NPY et la SP...), est à la recherche de peptides ayant une activité forte au niveau cutané.
Aussi est-il logique de voir de nombreuses études sur des séquences peptidiques essayant de mimer l'activité du collagène. Ces séquences essayent de se rapprocher soit d'une région spécifique du collagène, soit de sa forme très particulière.

Ainsi, DE 43 44 418 A1 décrit des peptides dérivés de l'hydrolyse du collagène et ayant une action anti-âge sur la peau. Spécifiquement sont décrits les tri-peptides Gly-His-Lys et Gly-Asp-Ser.

On constate donc que subsiste le besoin d'un nouveau dérivé du collagène, ayant une action sur l'adhésion des cellules cutanées.

Or, la demanderesse a trouvé, de façon surprenante et inattendue, qu'une quantité efficace d'un peptide de séquence (Gly-Pro-Gln)ₙ-NH₂ a une action sur l'adhésion cellulaire.

A la connaissance de la demanderesse, il n'a jamais été décrit dans l'art antérieur l'utilisation d'un peptide de séquence (Gly-Pro-Gln)ₙ-NH₂ en cosmétique.

Ainsi, l'invention a pour objet premier une composition cosmétique ou dermatologique, comprenant une quantité efficace de peptides de séquence (Gly-Pro-Gln)ₙ-NH₂ dans laquelle n est compris entre 1 et 3, et où les acides aminés peuvent être sous la forme L, D ou DL ; pour son application comme médicament dans le traitement curatif et/ou préventif des manifestations cutanées du vieillissement d'origine physiologique et solaire.

Par la suite, le terme « adhésion entre les cellules cutanées » doit s'entendre d'une part par l'adhésion entre les cellules cutanées et la matrice extracellulaire, et d'autre part par l'adhésion entre les cellules cutanées.

L'invention se rapporte encore à l'utilisation cosmétique non-thérapeutique d'une quantité efficace de peptides de séquence (Gly-Pro-Gln)ₙ-NH₂ dans laquelle n est compris entre 1 et 3, et où les acides aminés peuvent être sous la forme L, D ou DL ; pour traiter de manière curative et/ou préventive les manifestations cutanées du vieillissement (d'origine physiologique et solaire) et améliorer l'aspect de la peau.

Selon un mode de réalisation actuellement préféré de l'invention, le peptide précité possède la séquence (Gly-Pro-Gln)₂-NH₂.

Les peptides de l'invention peuvent être modifiés par acylation sur leur fonction N-terminale.

Les peptides de l'invention peuvent être obtenus soit par synthèse chimique ou enzymatique à partir des acides aminés constitutifs ou de leurs dérivés.

Les peptides de l'invention peuvent aussi être obtenus par hydrolyse ménagée de protéines naturelles (animale ou végétale).

Les peptides de l'invention peuvent enfin être obtenus par biotechnologie (utilisation d'un micro-organisme modifié ou non par génie génétique).

L'homme de l'art, connaissant le métier de l'extraction et de la purification des protéines et des peptides, pourra aisément envisager d'autres procédés d'obtention des peptides décrits par la demanderesse.

Selon un autre mode de réalisation avantageux de l'invention, le peptide précité est préalablement solubilisé dans un ou plusieurs solvants cosmétiquement ou pharmaceutiquement acceptables comme l'eau, le propylène glycol, le butylène glycol, les diglycols éthoxylés ou propoxylés, l'éthanol, le propanol ou l'isopropanol.

Selon encore un autre mode de réalisation avantageux de l'invention, le peptide précité est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

Pour donner un ordre de grandeur, dans la présente invention, le peptide de séquence (Gly-Pro-Gln)ₙ-NH₂ est utilisé en une concentration représentant de 0,0005 à 500 ppm (p/p) et préférentiellement en une concentration représentant de 0,05 à 50 ppm (p/p).

Pour donner un ordre de grandeur, dans la présente invention, le peptide de séquence (Gly-Pro-Gln)₂-NH₂ est utilisé en une concentration représentant de 0,0005 à 500 ppm (p/p) et préférentiellement en une concentration représentant de 0,05 à 50 ppm (p/p).

Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les poils ou les cheveux.
Ces compositions pourront notamment se présenter sous forme de crèmes, émulsions huile-dans-eau, eau-dans-huile ou émulsions multiples, solutions, suspensions, ou encore poudres, adaptées à une application sur la peau, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, de façon connue, les adjuvants nécessaires à leur formulation, tels que solvants, épaississants, diluants, anti-oxydants, colorants, filtres, pigments, charges, conservateurs, parfums, absorbeurs d'odeur. Dans tous les cas, ces adjuvants, ainsi que leurs proportions, seront choisis de manière à ne pas nuire aux propriétés recherchées dans l'invention. Ces adjuvants peuvent, par exemple, correspondre à 0,01 à 20 % du poids total de la composition.
Lorsque la composition de l'invention est une émulsion, la phase grasse peut représenter de 5 à 80 % en poids et de préférence de 5 à 50 % en poids par rapport au poids total de la composition. Les émulsionnants et coémulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme de métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Aussi, un autre objet de l'invention consiste en une composition cosmétique ou dermatologique, caractérisée en ce qu'elle contient, dans un milieu cosmétiquement ou dermatologiquement acceptable, un peptide de séquence (Gly-Pro-Gln)ₙ-NH₂ et préférentiellement un peptide possédant la séquence (Gly-Pro-Gln)₂-NH₂.

Dans les compositions de l'invention, le peptide de séquence (Gly-Pro-Gln)ₙ-NH₂ est utilisé en une concentration représentant de 0,0005 à 500 ppm (p/p) et préférentiellement en une concentration représentant de 0,05 à 50 ppm (p/p).

Dans les compositions de l'invention, le peptide de séquence (Gly-Pro-Gln)₂-NH₂ est utilisé en une concentration représentant de 0,0005 à 500 ppm (p/p) et préférentiellement en une concentration représentant de 0,05 à 50 ppm (p/p).

L'invention concerne enfin un procédé cosmétique non-thérapeutique pour le traitement des manifestations du vieillissement, consistant à appliquer, sur la peau ou les cheveux, la composition telle que définie précédemment.

D'autres avantages et caractéristiques de l'invention apparaîtront mieux à la lecture des exemples donnés à titre illustratif et non limitatif.

### Exemple 1 - Etude de la stabilité du peptide (Gly-Pro-Gln)₂-NH₂

Le peptide (Gly-Pro-Gln)₂-NH₂ a été analysé à une concentration de 10⁻⁴ M par HPLC sur une colonne de type C18, et avec un gradient linéaire eau/TFA 0,1% - acétonitrile/TFA 0,1%. Aucune dégradation du peptide n'a été observée après 24 heures à 25°C, 37°C et 60°C. De plus, après 8 jours à 25°C, le peptide ne présente toujours pas de dégradation.

Des fibroblastes et des kératinocytes humains ont été cultivés pendant 24 heures à 37°C et à 5 % de CO₂. Pendant cette période, les cellules peuvent libérer, dans le milieu de culture, des enzymes. Par la suite, le milieu de culture est retiré pour être mis en présence du peptide. Après 24 heures, le peptide ne présente pas de dégradation.

Lorsque le peptide appliqué sur des fibroblastes et kératinocytes en culture, un dosage HPLC du milieu de culture révèle que la concentration en peptide décroît rapidement en quelques heures.
Ces données, prises dans leur ensemble, suggèrent la possibilité d'une pénétration du peptide dans la cellule. Cette hypothèse est ensuite confortée par les tests d'efficacité exposés dans l'exemple 2.

### Exemple 2 - Effets du peptide de l'exemple 1 sur l'adhésion cellulaire

L'étude est réalisée dans des micro-plaques de 96 puits sur des kératinocytes cultivés dans un incubateur à 37°C et à 5 % de CO₂.

Selon les puits, différents peptides de 3 à 25 acides aminés sont incubés à une concentration de 35 ppm. Les contrôles négatifs ne contiennent pas de peptide et les contrôles positifs sont effectués avec du collagène I à une concentration de 60 ppm.

Après 3 heures les puits sont totalement remplis de milieu, hermétiquement fermés, retournés et agités sur un agitateur tridimensionnel pendant 20 minutes. Puis les plaques sont vidées du milieu et ensuite le milieu restant est aspiré. 100 µl de MTT à 1 mg/ml sont ajoutés par puits et laissés pendant 3 heures à 37°C et à 5 % CO₂. La solution est enfin retirée, puis 100 µl de DMSO sont ajoutés et la lecture est faite à 560 nm contre 630 nm. Pour la plupart des peptides et pour les contrôles sans peptide une densité optique de 0,49 UDO est obtenue à 540 nm.

Pour le collagène I testé à 60 ppm, la densité optique est de 0,68 UDO.

Pour le peptide de l'exemple 1 (Gly-Pro-Gln)₂-NH₂ testé à 5 ppm, la densité optique est de 0,62 UDO.

En conclusion, après seulement 3 heures de contact avec les kératinocytes, le peptide de séquence (Gly-Pro-Gln)₂-NH₂ apporte une bonne adhésion cellulaire, semblable à celle du collagène de type I, bien qu'utilisé en concentration plus faible que le collagène.

### Exemple 3 - Préparation de compositions

Ces compositions ont été obtenues par simple mélange des différents composants. Les quantités indiquées sont données en pourcentage de poids.

**1 - Emulsion huile dans eau**

| Phase huileuse : | |
|---|---|
| ▪Montanov 68 (Cetearyl Alcohol and Cetearyl Glucoside) | 5.00 % |
| ▪Huile de Jojoba | 5.00 % |
| ▪Huile de Vaseline | 5.00 % |
| ▪Isopropyl Palmitate | 7.00 % |

| Phase aqueuse : | |
|---|---|
| ▪Glycérine | 5.00 % |
| ▪Allantoïne | 0.10 % |
| ▪Peptide de l'exemple 1 | 5.00 % |
| ▪Sepigel 305 (Polyacrylamide and C13-14 Isoparaffin and Laureth-7) | 0.30 % |
| ▪Conservateur | 0.50 % |
| ▪Parfum | 0.50 % |
| ▪Eau | qsp 100 % |

**2 - Gel**

| | |
|---|---|
| ▪Carbopol Ultrez 10 (sol. à 2%) | 25.00 % |
| ▪Triéthanolamine | 0.50 % |
| ▪Peptide de l'exemple 1 | 1.0 % |
| ▪Conservateur | 0.20 % |
| ▪EDTA (séquestrant) | 0.10 % |
| ▪Parfum | 0.50 % |
| ▪Eau | qsp 100 % |

**3 - Lotion**

| | |
|---|---|
| ▪Mono Propylène Glycol | 1.00 % |
| ▪Allantoïne | 0.30 % |
| ▪Glycérine | 1.00 % |
| ▪Cetiol HE (PEG-7 Glyceryl Cocoate) | 1.00 % |
| ▪Peptide de l'exemple 1 | 1 ppm |
| ▪Conservateur | 0.20 % |
| ▪Parfum | 0.50 % |
| ▪Eau | qsp 100 % |

## Revendications

1. Composition comprenant une quantité efficace de peptides de séquence (Gly-Pro-Gln)ₙ-NH₂, dans laquelle n est compris entre 1 et 3 et où les acides aminés peuvent être sous la forme L, D ou DL ; pour son application comme médicament dans le traitement curatif et/ou préventif des manifestations cutanées du vieillissement d'origine physiologique et solaire.

2. Utilisation cosmétique non-thérapeutique d'une quantité efficace de peptides de séquence (Gly-Pro-Gln)ₙ-NH₂, dans laquelle n est compris entre 1 et 3 et où les acides aminés peuvent être sous la forme L, D ou DL ; pour traiter de manière curative et/ou préventive les manifestations cutanées du vieillissement d'origine physiologique et solaire.

3. Utilisation cosmétique non-thérapeutique d'une quantité efficace de peptides de séquence (Gly-Pro-Gln)ₙ-NH₂, dans laquelle n est compris entre 1 et 3 et où les acides aminés peuvent être sous la forme L, D ou DL ; pour améliorer l'aspect de la peau.

4. Utilisation non-thérapeutique selon la revendication 2 ou 3 **caractérisée en ce que** le peptide possède la séquence (Gly-Pro-Gln)₂-NH₂.

5. Utilisation non-thérapeutique selon l'une quelconque des revendications 2 à 4 **caractérisée en ce que** le peptide est modifié par acylation sur sa fonction N-terminale.

6. Utilisation non-thérapeutique selon l'une quelconque des revendications 2 à 5 **caractérisée en ce que** le peptide est obtenu par synthèse chimique ou enzymatique à partir des acides aminés constitutifs ou de leurs dérivés ; ou est obtenu par hydrolyse ménagée de protéines naturelles ; ou bien est obtenu par biotechnologie.

7. Utilisation non-thérapeutique selon l'une quelconque des revendications 2 à 6, **caractérisée en ce que** le peptide est préalablement solubilisé dans un ou plusieurs solvants cosmétiquement ou pharmaceutiquement acceptables comme l'eau, le propylène glycol, le butylène glycol, les diglycols éthoxylés ou propoxylés, l'éthanol, le propanol ou l'isopropanol.

8. Utilisation non-thérapeutique selon l'une quelconque des revendications 2 à 7, **caractérisée en ce que** le peptide est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

9. Utilisation non-thérapeutique selon l'une quelconque des revendications 2 à 8, **caractérisée en ce que** le peptide est utilisé en une concentration représentant de 0,0005 à 500 ppm (p/p) et préférentiellement en une concentration représentant de 0,05 à 50 ppm (p/p).

10. Composition cosmétique ou dermatologique, **caractérisée en ce qu'**elle contient, dans un milieu cosmétiquement ou dermatologiquement acceptable, au moins un peptide de séquence (Gly-Pro-Gln)ₙ-NH₂ tel que défini selon l'une quelconque des revendications 1 à 9.

11. Composition cosmétique selon la revendication 10 **caractérisée en ce qu'**elle contient au moins un peptide de séquence (Gly-Pro-Gln)₂-NH₂.

12. Composition selon la revendication 10 ou 11, **caractérisée en ce que** le peptide est utilisé à une concentration représentant de 0,0005 à 500 ppm (p/p) et préférentiellement à une concentration représentant de 0,05 à 50 ppm (p/p).

13. Procédé cosmétique non-thérapeutique pour le traitement des manifestations du vieillissement, consistant à appliquer, sur la peau ou les cheveux, la composition telle que définie selon l'une quelconque des revendications 10 à 12.

## Patentansprüche

1. Zusammensetzung, umfassend eine wirksame Menge von Peptiden mit der Sequenz (Gly-Pro-Gln)ₙ-NH₂, in der n zwischen 1 und 3 liegt, und wobei die Aminosäuren die Form L, D oder DL aufweisen können; für ihre Verwendung als Medikament bei der heilenden und/oder vorbeugenden Behandlung von Alterungsmanifestationen der Haut physiologischen Ursprungs oder durch Sonneneinwirkung.

2. Kosmetische, nicht therapeutische Verwendung einer wirksamen Menge von Peptiden mit der Sequenz (Gly-Pro-Gln)ₙ-NH₂, in der n zwischen 1 und 3 liegt, und wobei die Aminosäuren die Form L, D oder DL aufweisen können; um auf heilende und/oder vorbeugende Weise die Alterungsmanifestationen der Haut physiologischen Ursprungs oder durch Sonneneinwirkung zu behandeln.

3. Kosmetische, nicht therapeutische Verwendung einer wirksamen Menge von Peptiden mit der Sequenz (Gly-Pro-Gln)ₙ-NH₂, in der n zwischen 1 und 3 liegt und wobei die Aminosäuren die Form L, D oder DL aufweisen können; um das Aussehen der Haut zu verbessern.

4. Nicht therapeutische Verwendung nach Anspruch 2 oder 3, **dadurch gekennzeichnet, dass** das Peptid die Sequenz (Gly-Pro-Gln)₂-NH₂ aufweist.

5. Nicht therapeutische Verwendung nach einem der Ansprüche 2 bis 4, **dadurch gekennzeichnet, dass** das Peptid durch Acylierung auf seiner N-terminalen Funktion modifiziert wird.

6. Nicht therapeutische Verwendung nach einem der Ansprüche 2 bis 5, **dadurch gekennzeichnet, dass** das Peptid durch chemische oder enzymatische Synthese aus den konstitutiven Aminosäuren oder ihren Derivaten erhalten wird; oder durch gelenkte Hydrolyse von natürlichen Proteinen erhalten wird; oder auch durch Biotechnologie erhalten wird.

7. Nicht therapeutische Verwendung nach einem der Ansprüche 2 bis 6, **dadurch gekennzeichnet, dass** das Peptid zuvor in einem oder in mehreren kosmetisch oder pharmazeutisch annehmbaren Lösemitteln wie z.B. Wasser, Propylenglykol, Butylenglykol, ethoxylierten oder propoxylierten Diglykolen, Ethanol, Propanol oder Isopropanol solubilisiert wird.

8. Nicht therapeutische Verwendung nach einem der Ansprüche 2 bis 7, **dadurch gekennzeichnet, dass** das Peptid vorher in einem kosmetischen oder pharmazeutischen Vektor wie z.B. den Liposomen solubilisiert wird, oder auf pulverförmigen organischen Polymeren, mineralischen Trägern wie z.B. Talkmineralien und Bentoniten adsorbiert wird und insbesondere in jedem kosmetisch oder pharmazeutisch annehmbaren Vektor solubilisiert oder darauf fixiert wird.

9. Nicht therapeutische Verwendung nach einem der Ansprüche 2 bis 8, **dadurch gekennzeichnet, dass** das Peptid in einer Konzentration verwendet wird, die von 0,0005 bis 500 ppm (p/p) darstellt und vorzugsweise in einer Konzentration, die von 0,05 bis 50 ppm (p/p) darstellt.

10. Kosmetische oder dermatologische Zusammensetzung, **dadurch gekennzeichnet, dass** sie, in einem kosmetisch oder dermatologisch annehmbaren Medium, mindestens ein Peptid mit der Sequenz (Gly-Pro-Gln)ₙ-NH₂ enthält, wie nach einem der Ansprüche 1 bis 9 beschrieben.

11. Kosmetische Zusammensetzung nach Anspruch 10, **dadurch gekennzeichnet, dass** sie mindestens ein Peptid mit der Sequenz (Gly-Pro-Gln)₂-NH₂ enthält.

12. Kosmetische Zusammensetzung nach Anspruch 10 oder 11, **dadurch gekennzeichnet, dass** das Peptid in einer Konzentration verwendet wird, die von 0,0005 bis 500 ppm (p/p) darstellt und vorzugsweise in einer Konzentration, die von 0,05 bis 50 ppm (p/p) darstellt

13. Kosmetisches, nicht therapeutisches Verfahren zur Behandlung der Alterungsmanifestationen, die darin besteht, die Zusammensetzung, wie in einem der Ansprüche 10 bis 12 definiert, auf die Haut oder die Haare aufzubringen.

## Claims

1. Composition comprising a sufficient amount of peptides of sequence (Gly-Pro-Gln)ₙ-NH₂, wherein n ranges between 1 and 3 and wherein the amino acids can be in the form L, D or DL; for its application as a drug in the curative and/or preventive treatment of ageing skin symptoms of physiological and solar origin.

2. Cosmetic non-therapeutic use of a sufficient amount of peptides of sequence (Gly-Pro-Gln)ₙ-NH₂, wherein n ranges between 1 and 3 and wherein the amino acids can be in the form L, D or DL; to provide curative and/or preventive treatment for ageing skin symptoms of physiological and solar origin.

3. Cosmetic non-therapeutic use of a sufficient amount of peptides of sequence (Gly-Pro-Gln)ₙ-NH₂, wherein n ranges between 1 and 3 and wherein the amino acids can be in the form L, D or DL; to enhance skin appearance.

4. Non-therapeutic use according to claim 2 or 3 **characterised in that** the peptide has the sequence (Gly-Pro-Gln)ₙ-NH₂.

5. Non-therapeutic use according to any of claims 2 to 4 **characterised in that** the peptide is modified by acylation on the N-terminal function thereof.

6. Non-therapeutic use according to any of claims 2 to 5 **characterised in that** the peptide is obtained by chemical or enzymatic synthesis using constituting amino acids or the derivatives thereof; or is obtained by mild hydrolysis of natural protein; or is obtained by biotechnology.

7. Non-therapeutic use according to any of claims 2 to 6, **characterised in that** the peptide is solubilised beforehand in one or more cosmetically or dermatologically acceptable solvents such as water, glycol propylene, glycol butylene, ethoxylated or propoxylated diglycols, ethanol, propanol or isopropanol.

8. Non-therapeutic use according to any of claims 2 to 7, **characterised in that** the peptide is solubilised beforehand in a cosmetic or pharmaceutical vector such as liposomes or adsorbed on powdery organic polymers, mineral supports such as talcs and bentonites, and more generally solubilised in, or fixed on, any cosmetically or dermatologically acceptable vector.

9. Non-therapeutic use according to any of claims 2 to 8, **characterised in that** the peptide is used at a concentration representing from 0.0005 to 500 ppm (p/p) and preferentially at a concentration representing from 0.05 to 50 ppm (p/p).

10. Cosmetic or dermatological composition, **characterised in that** it contains, in a cosmetically or dermatologically acceptable medium, at least one peptide of sequence (Gly-Pro-Gln)ₙ-NH₂ such as defined according to any of claims 1 to 9.

11. Cosmetic composition according to claim 10 **characterised in that** it contains at least one peptide of sequence (Gly-Pro-Gln)ₙ-NH₂.

12. Composition according to claim 10 or 11, **characterised in that** the peptide is used at a concentration representing from 0.0005 to 500 ppm (p/p) and preferentially at a concentration representing from 0.05 to 50 ppm (p/p).

13. Non-therapeutic cosmetic method for the treatment of ageing skin symptoms, consisting in applying, on the skin or hair, the composition such as defined according to any of claims 10 to 12.
